# EUROPEAN PATENT APPLICATION

(11) **EP 4 316 430 A1**
(43) Date of publication of application: **07.02.2024**
(21) Application number: 22780272.5
(22) Date of filing: 18.03.2022
(51) Int. Cl.: A61F 2/966

(54) **INDWELLING DEVICE**

(30) Priority: 30.03.2021 JP 2021057476; 31.03.2021 JP 2021059197
(71) Applicant: SB-Kawasumi Laboratories, Inc., Kawasaki-shi, Kanagawa 210-8602 (JP); Sumitomo Bakelite Co., Ltd., Shinagawa-ku Tokyo 140-0002 (JP)
(72) Inventor: TAMAI, Yusuke, Kawasaki-shi, Kanagawa 210-8602 (JP); YAMAMOTO, Naoaki, Kawasaki-shi, Kanagawa 210-8602 (JP); EMI, Tomohiro, Kawasaki-shi, Kanagawa 210-8602 (JP)
(74) Representative: Müller-Boré & Partner Patentanwälte PartG mbB
(86) International application number: PCT/JP2022/012884
(87) International publication number: WO 2022/210057

(57) **Abstract**

A placement device 1 is a placement device for placing a stent graft 50 in a biological lumen, the placement device including: a sheath 11 in which the stent graft is accommodated; string-like converting means 14 that causes at least a portion of the stent graft in an axial direction to be maintained in a contracted state in a state in which the stent graft is released from the sheath and is able to convert the portion from the contracted state to an expanded state; and operating means 20 operated to place the stent graft in the biological lumen. The operating means has a first operating portion 20A for releasing the stent graft from the sheath, and a second operating portion 20B for releasing restraint of the portion of the stent graft by the converting means.

## Description

### Technical Field

The present invention relates to a placement device.

### Background Art

In the related art, a tubular indwelling tool such as a stent graft is known which is used for the treatment of an aneurysm or the like that occurs in a blood vessel wall. For example, an abdominal stent graft used to treat a lesion site of the abdominal aorta (for example, aortic aneurysm or aortic dissection) needs to be placed over an area from the abdominal aorta to the left and right common iliac arteries and thus generally has an inverted "Y" shape.

In the placement of a stent graft, after a placement device is introduced into a biological lumen and delivered to a lesion site, a tubular indwelling tool in a contracted state is released from a sheath, and the tubular indwelling tool expands to be placed in the biological lumen. Usually, the tubular indwelling tool expands in order from a distal side portion released from the sheath.

A placement device is also proposed in which a contracted state of a tubular indwelling tool is maintained even in a state of being released from a sheath and the restraint is released to transition to an expanded state by a predetermined operation, whereby the tubular indwelling tool is partially released and can be placed in a biological lumen (refer to Patent Document 1, for example).

### Prior Art Document

### Patent Document

[Patent Document 1] Japanese Patent No. 5408866

### Summary of Invention

### Technical Problem

As an example of a method of partially releasing a tubular indwelling tool to be placed in a biological lumen, there is a method of restraining a contracted state of a tubular indwelling tool using a string-like member. In this case, in order to release the restraint of the tubular indwelling tool, it is necessary to pull out the string-like member by pulling an operating device attached to the string-like member, and an operation of releasing the restraint of the tubular indwelling tool by the string-like member is complicated. In addition, in order to place the tubular indwelling tool in a biological lumen, it is necessary to perform an operation of releasing the tubular indwelling tool from a sheath. Therefore, there is a concern that the tubular indwelling tool cannot be properly placed in the biological lumen depending on an operator's skill.

An object of the present invention is to properly place a tubular indwelling tool in a biological lumen.

### Solution to Problem

A placement device according to the present invention is a placement device for placing a tubular indwelling tool in a biological lumen, the placement device including: a sheath in which the tubular indwelling tool is accommodated; string-like converting means that causes at least a portion of the tubular indwelling tool in an axial direction to be maintained in a contracted state in a state in which the tubular indwelling tool is released from the sheath and is able to convert the portion from the contracted state to an expanded state; and operating means operated to place the tubular indwelling tool in the biological lumen, in which the operating means has a first operating portion for releasing the tubular indwelling tool from the sheath, and a second operating portion for releasing restraint of the portion of the tubular indwelling tool by the converting means.

### Advantageous Effects of Invention

According to the present invention, a tubular indwelling tool can be properly placed in a biological lumen.

### Brief Description of Drawings

Fig. 1 is a perspective view showing an overall configuration of a placement device.
Fig. 2 is a view schematically showing a state in which a stent graft is placed in a blood vessel.
Fig. 3 is a schematic view showing an example of converting means.
Fig. 4 is a view showing an internal structure of operating means.
Fig. 5 is an exploded perspective view of a distal part of a first operating portion.
Fig. 6 is a cross-sectional view of the distal part of the first operating portion.
Fig. 7 is an exploded perspective view of a gearbox, a trigger, and a gear train.
Fig. 8 is an exploded perspective view of a second operating portion.
Fig. 9 is a cross-sectional view of the second operating portion.
Figs. 10A to 10C are views showing a change in a state of the stent graft during placement.
Figs. 11A and 11B are schematic views showing an operation of the operating means during the placement.
Fig. 12 is an exploded perspective view of a second operating portion according to a modification example.
Fig. 13 is a cross-sectional view of the second operating portion according to the modification example.
Fig. 14 is a partially cutaway cross-sectional perspective view of the second operating portion according to the modification example.

### Description of Embodiments

Hereinafter, an embodiment of the present invention will be described in detail with reference to the drawings. In the present embodiment, as an example of a placement device according to the present invention, a placement device 1 for placing a stent graft 50, which is a tubular indwelling tool, in a lesion site L of an abdominal aorta C with an aneurysm will be described.

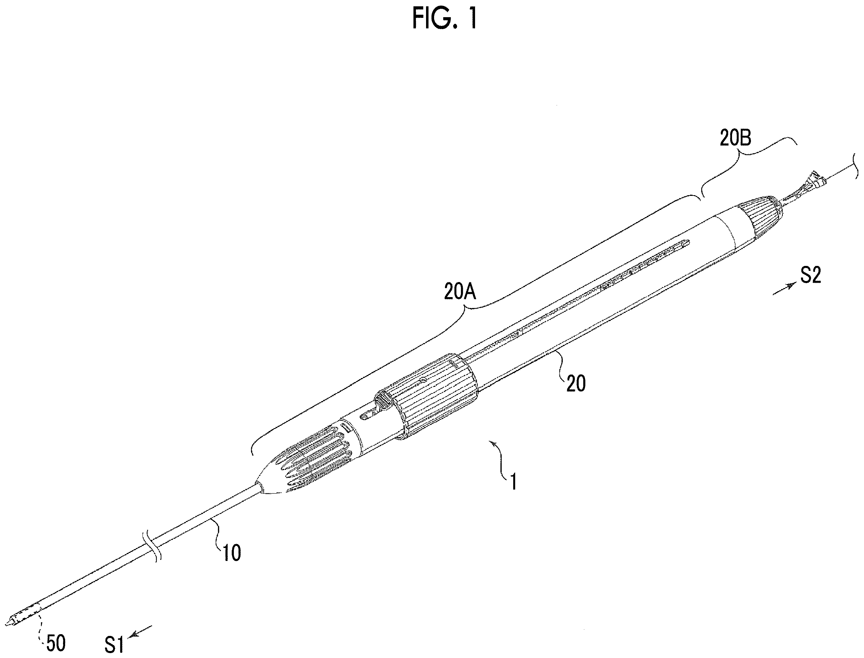

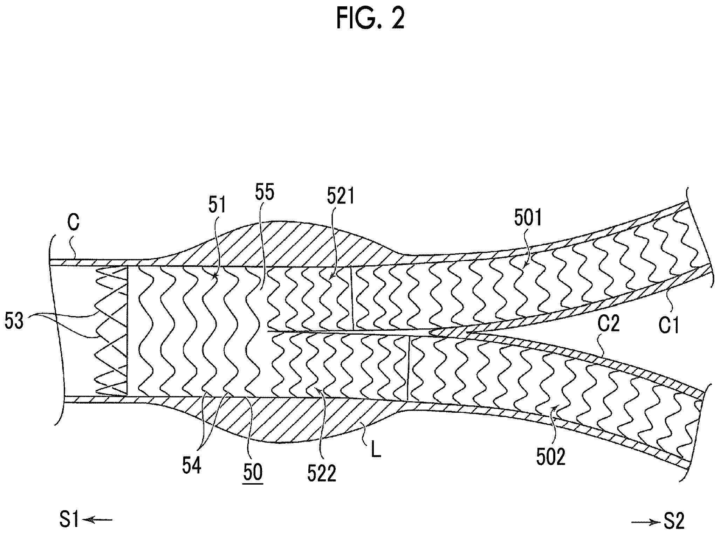

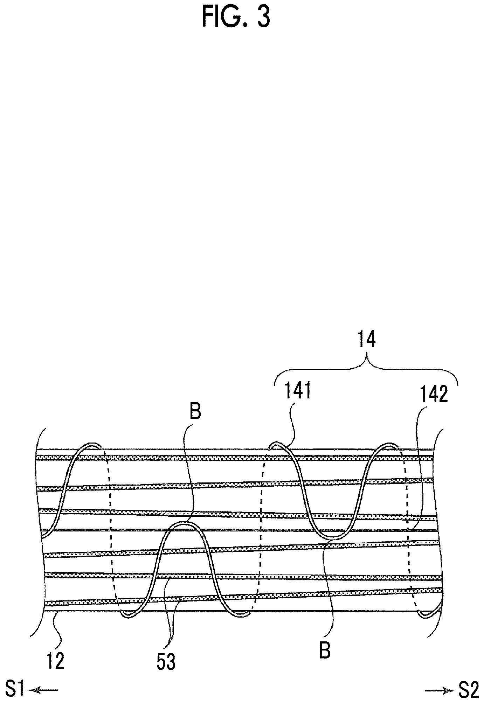

Fig. 1 is a perspective view showing an overall configuration of the placement device 1 according to the embodiment. Fig. 2 is a view schematically showing a state in which the stent graft 50 is placed in the abdominal aorta C. Fig. 3 is a schematic view showing an example of converting means 14.

First, a configuration of the stent graft 50 will be described with reference to Fig. 2.

The stent graft 50 is an example of a tubular indwelling tool, and is applied to expand a lesion site of the abdominal aorta C. The stent graft 50 includes a main body portion 51, a first branch portion 521 and a second branch portion 522 branching from an end portion of the main body portion 51 on a proximal side S2 (peripheral side), and a bare portion 53 disposed at an end portion of the main body portion 51 on a distal side S1 (central side). The first branch portion 521 and the second branch portion 522 have a smaller tube diameter than the main body portion 51, and are connected to the end portion of the main body portion 51 on the proximal side S2 so as to branch from the end portion into two portions. That is, the stent graft 50 has an overall "Y" shape. The stent graft 50 is placed in the abdominal aorta C such that the bare portion 53 is on an upstream (heart) side in a blood flow direction, and the first branch portion 521 and the second branch portion 522 are on a downstream side.

The first branch portion 521 and the second branch portion 522 are respectively connected to, for example, first and second extension stent grafts 501 and 502, and the first and second extension stent grafts 501 and 502 are placed in the left common iliac artery C1 and the right common iliac artery C2. Accordingly, the inflow of blood into the lesion site L can be suppressed without inhibiting the flow of blood from the upstream side to the downstream side of the branched blood vessel (abdominal aorta C).

The stent graft 50 is constituted by a skeleton 54 and a membrane 55. The skeleton 54 is sewn to the membrane 55, for example, by a suture (not shown).

The skeleton 54 is formed, for example, in a ring skeleton in which a single metal wire extends in a circumferential direction while being bent in a zigzag shape (Z shape) such that peak portions (bent portions on the distal side S1) and valley portions (bent portions on the proximal side S2) are alternately formed. The skeleton 54 is configured to be self-expandable from a contracted state of being contracted radially inward to an expanded state of being expanded radially outward. The skeleton 54 may be a laser-cut type formed by performing laser processing on a metallic cylindrical member.

Examples of a material forming the skeleton 54 include known metals or metal alloys represented by stainless steel, a nickel-titanium alloy (nitinol), and a titanium alloy. Also, for the skeleton 54, an alloy material having X-ray contrast properties may be used. In a case where the nickel-titanium alloy is used as the material of the skeleton 54, by adjusting the skeleton 54 into a shape in the expanded state and thereafter subjecting the skeleton 54 to a predetermined heat treatment, the skeleton 54 can memorize the shape in the expanded state. The skeleton 54 may be formed of a material other than metal (for example, ceramic or a resin).

The membrane 55 is a flexible membrane body that forms a blood flow path. Examples of a material for forming the membrane 55 include a fluororesin such as polytetrafluoroethylene (PTFE) and a polyester resin such as polyethylene terephthalate.

The main body portion 51, the first branch portion 521, and the second branch portion 522 have a tubular shape that defines a blood flow path. On a peripheral surface of the membrane 55 in each of the main body portion 51, the first branch portion 521, and the second branch portion 522, a plurality of the skeletons 54 are disposed at predetermined intervals along an axial direction (extension direction of the stent graft 50). The membrane 55 may be disposed on an outer peripheral surface side and on an inner peripheral surface side of the skeleton 54 with the skeleton 54 interposed therebetween, or may be disposed only on the outer peripheral surface or only on the inner peripheral surface of the skeleton 54.

The bare portion 53 has a function of generating friction with a blood vessel wall when the stent graft 50 is placed and suppressing positional deviation (migration) of the stent graft 50. A portion of the skeleton 54 of the bare portion 53 on the proximal side S2 is fixed to the membrane 55, and almost all portion thereof is exposed from the membrane 55. A fixing pin (reference numeral omitted) is provided in the skeleton 54 of the bare portion 53 so as to protrude radially outward. The fixing pin assists fixation of the bare portion 53 to a blood vessel by biting into the blood vessel wall.

Next, the configuration of the placement device 1 according to the embodiment will be described with reference to Fig. 1 and the like.

As shown in Fig. 1 and the like, the placement device 1 includes a catheter portion 10 that is introduced into a blood vessel, and operating means 20 that is operated when the stent graft 50 is placed into the blood vessel.

The catheter portion 10 has a sheath 11, an inner rod 12, a distal tip 13, the converting means 14, and the like. The stent graft 50 is mounted on an end portion of the inner rod 12 on the distal side S1 and is accommodated in the sheath 11.

The sheath 11 is, for example, a tubular member formed of a flexible material. Examples of the material forming the sheath 11 include a biocompatible synthetic resin (elastomer) selected from a fluororesin, a polyamide resin, a polyethylene resin, and a polyvinyl chloride resin, a resin compound in which other materials are mixed in the synthetic resins, a multilayer structure formed of the synthetic resins, and a composite of the synthetic resins and a metal wire.

The inner rod 12 is a shaft-like member longer than the sheath 11. Examples of a material for forming the inner rod 12 include various materials having appropriate hardness and flexibility, such as resins (plastics and elastomers) and metals. The inner rod 12 has an inner cavity into which a guide wire (not shown) is inserted.

Proximal sides S2 of the sheath 11 and the inner rod 12 are respectively connected to a first operating portion 20A and a second operating portion 20B. The distal tip 13 is disposed at a distal end of the inner rod 12.

The inner rod 12 is inserted into the sheath 11 and is disposed to extend to a distal side S1 of the sheath 11 in a state in which the stent graft 50 is mounted on the end portion of the inner rod 12 on the distal side S1. The sheath 11 and the inner rod 12 are configured to be relatively movable in an axial direction of the sheath 11 by operating the operating means 20 (mainly the first operating portion 20A).

The stent graft 50 mounted on the inner rod 12 is restrained by the converting means 14 so that it is difficult for a portion (for example, the bare portion 53) of the stent graft 50 to expand. Specifically, the converting means 14 has a string-like member, causes at least a portion of the stent graft 50 to be maintained in a contracted state in a state in which the stent graft 50 is released from the sheath 11, and converts at least the portion from the contracted state to an expanded state by an operation of the second operating portion 20B.

For example, as shown in Fig. 3, the converting means 14 is constituted by a string-like restraining string 141 and a holding wire 142. The restraining string 141 is wound around an outer peripheral surface of the stent graft 50, and the holding wire 142 is engaged with the restraining string 141 wound around the outer peripheral surface of the stent graft 50 to hold the wound state of the restraining string 141. For example, the converting means 14 is mounted on the stent graft 50 mounted on the inner rod 12 so as to contract the stent graft 50 in a radial direction.

The restraining string 141 and the holding wire 142 are formed of, for example, a material having predetermined strength and rigidity, and for example, a suture such as nylon fibers and fluorine fibers, a nickel-titanium alloy, a thin metal wire made of stainless steel, and a string-like member made of a resin can be applied. The restraining string 141 and the holding wire 142 are preferably formed of different materials in order to improve slipperiness when pulled out.

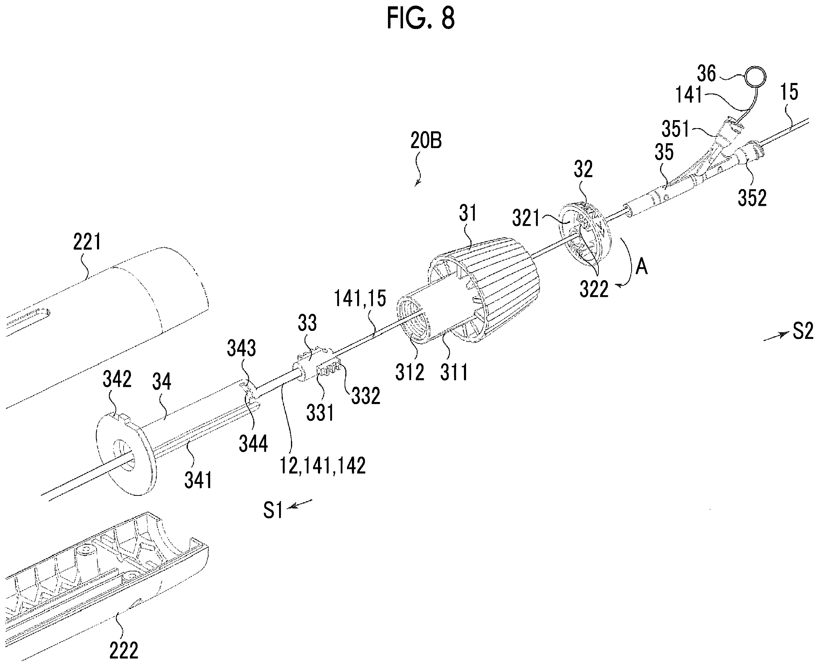

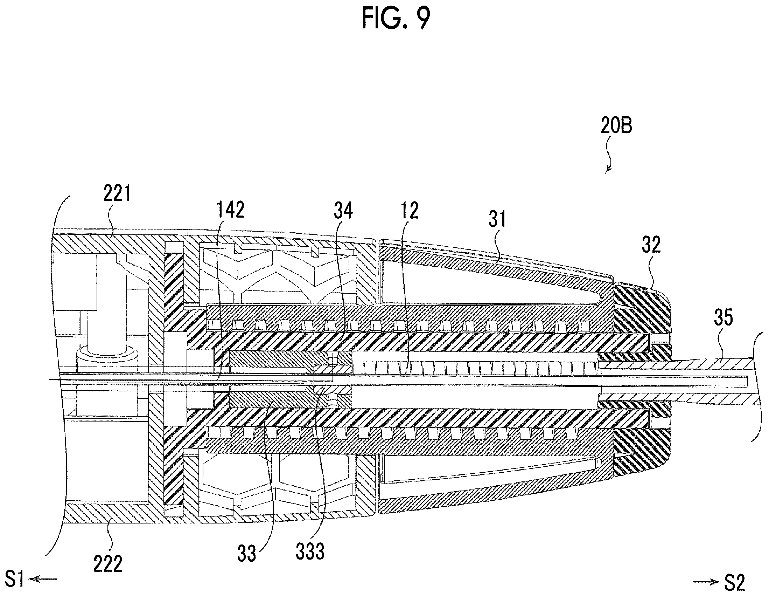

The stent graft 50 is held in the contracted state by portions of the restraining string 141 and the holding wire 142 on the distal side S1. An end portion of the restraining string 141 on the proximal side S2 is pulled out to the outside from one insertion hole 351 of a Y connector 35 and is connected to an operating device 36 (see Fig. 8) . The restraining string 141 is pulled out by an operation of pulling the operating device 36. An end portion of the holding wire 142 on the proximal side S2 is connected to a fixing terminal 33 of the second operating portion 20B (see Fig. 9). The holding wire 142 is moved to the proximal side S2 by operating a rear handle 31 of the second operating portion 20B such that the engaged state with the restraining string 141 is released.

The restraining string 141 is wound in the circumferential direction around an outer peripheral surface of the portion (for example, the bare portion 53) of the stent graft 50, and is bent and wound in an opposite direction for each rotation. On the other hand, the holding wire 142 is disposed along an axial direction of the stent graft 50 and is engaged with a bent portion B formed in the restraining string 141. That is, the restraining string 141 is wound in such a manner that the restraining string 141 cannot hold the wound state by itself, and is held so as not to fall off by being engaged with the holding wire 142. Therefore, when the holding wire 142 is moved to the proximal side S2 by rotating the rear handle 31 and the engaged state between the restraining string 141 and the holding wire 142 is released, the restraining string 141 naturally falls off the stent graft 50. As a result, the portion of the stent graft 50 is released from the contracted state, enters a state of being expandable in the radial direction, and transitions to the expanded state.

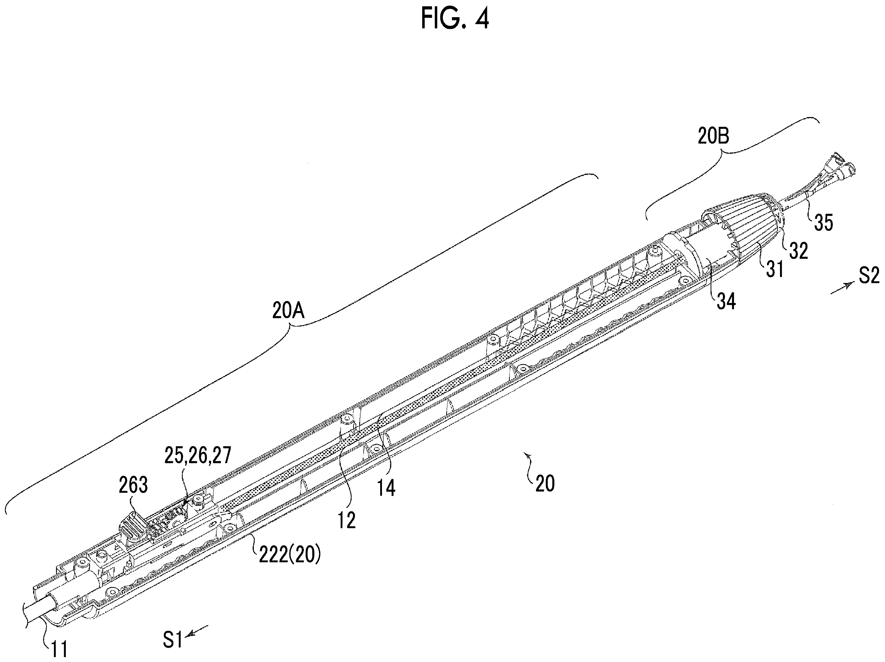

The operating means 20 is provided on a proximal side S2 of the catheter portion 10. The operating means 20 includes the first operating portion 20A and the second operating portion 20B. Fig. 4 is a view showing an internal structure of the operating means 20. Fig. 4 shows a state of the operating means 20 with a front cover 21, an upper cover 221, and a front handle 23 removed.

The first operating portion 20A is an operating portion for releasing the stent graft 50 from the sheath 11 by relatively moving the sheath 11 and the inner rod 12 in the axial direction. The first operating portion 20A has a sheath fixing portion that fixes the sheath 11, a moving mechanism that moves the sheath fixing portion in the axial direction, and covers 21 and 22 that accommodate the sheath fixing portion. The sheath fixing portion is configured to include a gearbox 25 and the like, and the moving mechanism is configured to include a gear train 27, a rack 28, the front handle 23, and the like.

The second operating portion 20B is an operating portion for releasing the restraint of the stent graft 50 by the converting means 14 by moving the string-like converting means 14 to the proximal side S2. In addition, an end portion of the inner rod 12 on the proximal side S2 is connected to the second operating portion 20B, and the sheath 11 can be moved in the axial direction by operating the first operating portion 20A while an axial position of the inner rod 12 is fixed.

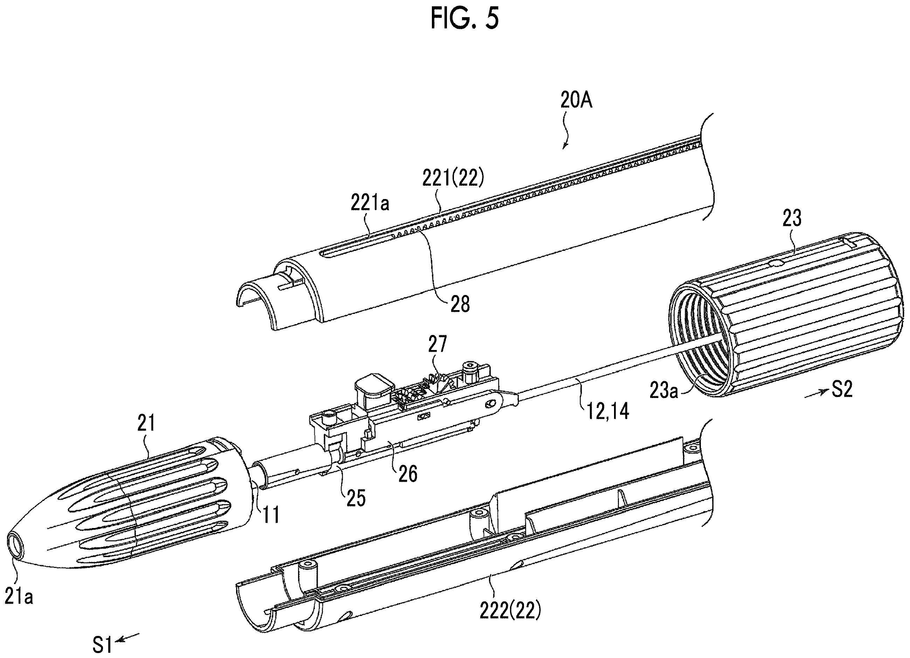

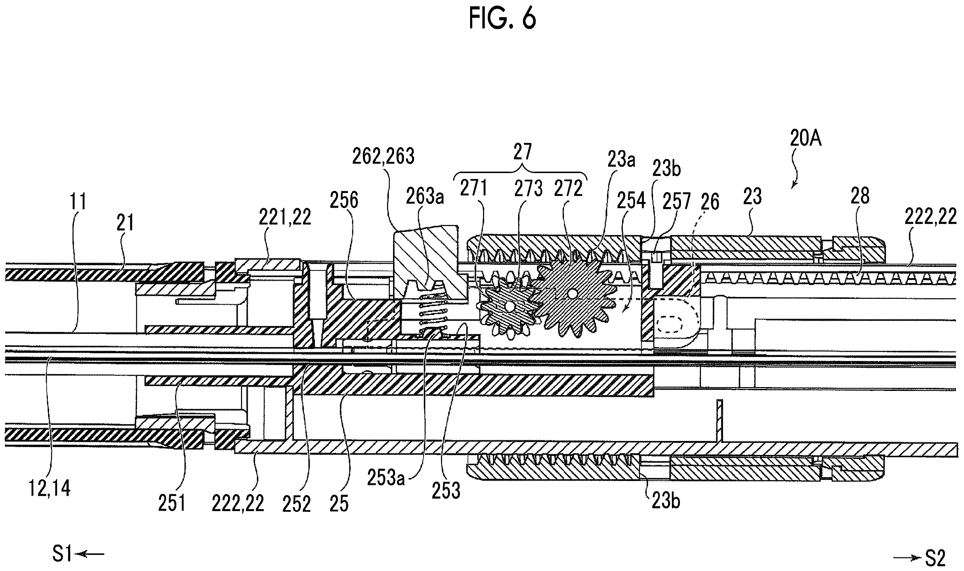

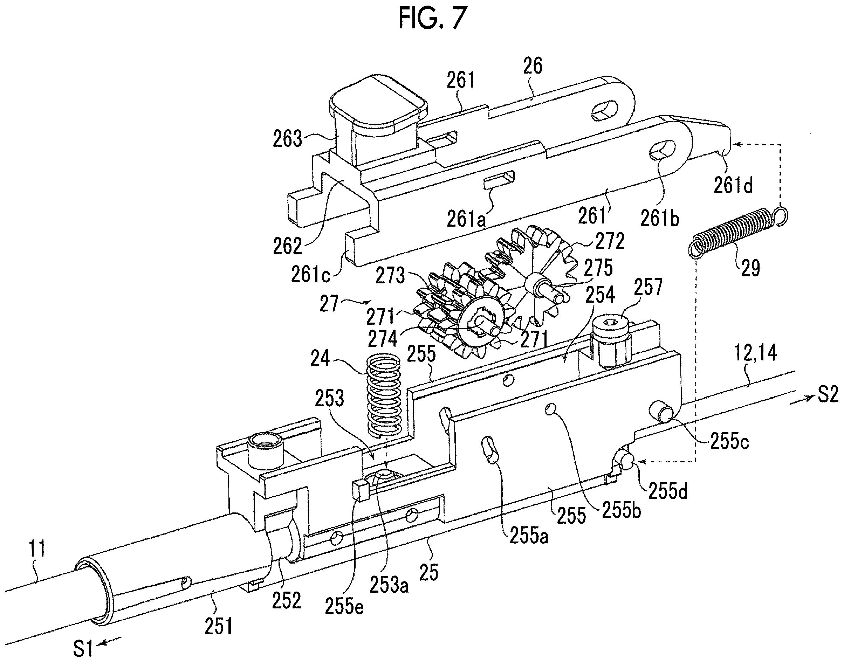

Fig. 5 is an exploded perspective view of the first operating portion 20A. Fig. 6 is a cross-sectional view of the first operating portion 20A. Fig. 7 is an exploded perspective view of the gearbox 25, a trigger 26, and the gear train 27.

As shown in Fig. 5 and the like, the first operating portion 20A includes the front cover 21, a cover main body 22, the front handle 23, the gearbox 25, the trigger 26, the gear train 27, the rack 28, and the like.

The front cover 21 has a hollow semi-fusiform shape and is attached to a distal side S1 of the cover main body 22. The front cover 21 has an opening 21a at a tip end portion thereof through which the catheter portion 10 is inserted.

The cover main body 22 has a cylindrical shape. Further, the cover main body 22 has a divided structure including the upper cover 221 and a lower cover 222 having a semi-cylindrical shape. The cover main body 22 accommodates therein the gearbox 25, the trigger 26, the gear train 27, the rack 28, and the like. The second operating portion 20B is attached to an end portion of the cover main body 22 on the proximal side S2.

The upper cover 221 has an oblong slit 221a along the axial direction. A second gear 272 of the gear train 27 protrudes outward from the slit 221a and meshes with a worm 23a of the front handle 23. A button 263 of the trigger 26 protrudes outward from the slit 221a and can be operated by an operator. An axial length of the slit 221a is set to be longer than a movable length of the gearbox 25 (sheath fixing portion).

The rack 28 is disposed on an inner peripheral surface of the upper cover 221 along both peripheral edges along the axial direction of the slit 221a. In addition, the rack 28 is formed integrally with the upper cover 221. An axial length of the rack 28 corresponds to the movable length of the gearbox 25 (sheath fixing portion).

The front handle 23 has a cylindrical shape and is fitted to an outer peripheral surface of the cover main body 22. An inner peripheral surface of the front handle 23 has the worm 23a (screw gear). The worm 23a meshes with the second gear 272 of the gear train 27. An engagement groove 23b that engages with a stopper 257, which will be described later, is provided on the inner peripheral surface of the front handle 23 at a position of the worm 23a on the proximal side S2 along the circumferential direction. The engagement groove 23b is a groove of a predetermined depth provided from the inner peripheral surface of the front handle 23 toward an outer surface thereof. A part of the engagement groove 23b opens to the outer surface of the front handle 23.

The gearbox 25 has a sheath connection portion 251, a rod insertion portion 252, a spring holding portion 253, a gear accommodation portion 254, a restriction portion 256, and the stopper 257. In addition, the gearbox 25 forms a part of the sheath fixing portion that fixes the sheath 11.

The sheath connection portion 251 has a cylindrical shape and fixes a proximal end of the sheath 11. In addition, the sheath connection portion 251 is provided at an end portion of the gearbox 25 on the distal side S1.

The rod insertion portion 252 is provided on a proximal side S2 of the sheath connection portion 251. The inner rod 12 and the converting means 14 exposed from the proximal end of the sheath 11 are inserted through the rod insertion portion 252.

The spring holding portion 253 holds a first spring 24. The spring holding portion 253 has a protruding portion 253a protruding upward. The gear accommodation portion 254 accommodates the gear train 27.

The spring holding portion 253 and the gear accommodation portion 254 are open at the top. The spring and the gear accommodation portion 254 are formed by two side plates 255 directed in the axial direction. The side plates 255 and 255 each have a through-hole 255a, a bearing hole 255b, a trigger connection portion 255c, and a locking portion 255e. One side plate 255 (the side plate 255 on the front side in Fig. 7) has a spring attachment portion 255d.

The through-hole 255a is an oblong hole extending obliquely downward toward the proximal side S2. A first gear shaft 274 is inserted through the through-hole 255a. The bearing hole 255b supports a second gear shaft 275. The trigger connection portion 255c is engaged with an engagement hole 261b of the trigger 261. One end of a second spring 29 is connected to the spring attachment portion 255d. The second spring 29 is configured as, for example, a compression coil spring. The locking portion 255e locks a locking piece 261c of the trigger 26.

The restriction portion 256 is provided at a center in a width direction along the axial direction on the distal side S1 of the spring holding portion 253. The restriction portion 256 is positioned below a connection portion 262 of the trigger 26 and restricts the button 263 from being pressed directly downward.

The stopper 257 is a screw-like member provided upward on the proximal side S2 of the gear accommodation portion 254. The stopper 257 is attached to a screw hole (reference numerals omitted) of the gearbox 25 via an opening connected to the engagement groove 23b of the front handle 23, and is configured to be able to adjust an upper surface position of the stopper 257 by using a jig. As shown in Fig. 6, by setting the upper surface position of the stopper 257 to be higher than the inner peripheral surface of the front handle 23, the front handle 23 can be prevented from falling off the gear train 27, and the front handle 23 and the gearbox 25 can be moved integrally along the axial direction.

The trigger 26 has two side plates 261 and 261 directed in the axial direction. The side plates 261 and 261 are connected to each other with the connection portion 262 on the distal side S1. The button 263 is disposed on the connection portion 262.

The side plates 261 and 261 each have a bearing hole 261a, the engagement hole 261b, the locking piece 261c, and a spring attachment portion 261d.

The bearing hole 261a and the engagement hole 261b have an oblong shape along the axial direction. The first gear shaft 274 is inserted through the bearing hole 261a. The trigger connection portion 255c of the gearbox 25 is engaged with the engagement hole 261b. The locking piece 261c is locked to the locking portion 255e of the gearbox 25. The other end of the second spring 29 is connected to the spring attachment portion 261d.

The button 263 has a protruding portion 263a protruding downward. The first spring 24 is mounted between the protruding portion 253a of the spring holding portion 253 and the protruding portion 263a of the button 263. The first spring 24 is configured as, for example, a compression coil spring.

The trigger 26 is attached to the gearbox 25 in a state of being biased obliquely upward by the first spring 24. Here, the side plate 261 of the trigger 26 is positioned outside the side plate 255 of the gearbox 25, and the locking piece 261c is positioned below the locking portion 255e. The trigger connection portion 255c of the gearbox 25 is fitted into the engagement hole 261b of the trigger 26, and the locking piece 261c of the trigger 26 is locked to the locking portion 255e of the gearbox 25, whereby the gearbox 25 and the trigger. 26 are integrated and held in a biased state.

That is, the sheath fixing portion is configured to include the trigger 26 that supports a first gear 271, the gearbox 25 that supports the second gear 272, and the first spring 24 that is interposed between the trigger 26 and the gearbox 25 and biases the trigger 26 in a direction in which the first gear 271 meshes with the rack 28 to hold the meshing state. The gearbox 25 also supports the first gear 271 together with the second gear 272.

The gear train 27 has the first gear 271, the second gear 272, and a third gear 273. The first gear 271 is a pinion gear that meshes with the rack 28. In addition, two first gears 271 are provided with the third gear 273 interposed therebetween. The second gear 272 is a worm wheel that meshes with the worm 23a of the front handle 23. The third gear 273 is a relay gear that transmits rotation of the second gear 272 to the first gear 271. The third gear 273 is fixed to the first gear shaft 274 similarly to the first gear 271.

The first gear shaft 274 to which the first gear 271 and the third gear 273 are fixed is inserted through the bearing hole 261a of the trigger 26 via the through-hole 255a of the gearbox 25. The second gear shaft 275 to which the second gear 272 is fixed is inserted through the bearing hole 255b of the gearbox 25.

In the first operating portion 20A, when the front handle 23 is rotated in one direction about the axial direction, the second gear 272 rotates via the worm 23a. The rotation of the second gear 272 is transmitted to the first gear 271 via the third gear 273. The rotation of the first gear 271 is converted by the rack 28 into linear motion along the axial direction. Since the rack 28 is fixed to the cover main body 22, the gearbox 25 having the gear train 27 moves in the axial direction.

Since the sheath 11 is fixed to the gearbox 25 and the inner rod 12 is fixed to the fixing terminal 33 in a rod guide shaft 34 fixed to the cover main body 22, the sheath 11 moves in the axial direction with respect to the inner rod 12. Accordingly, the stent graft 50 disposed on the inner rod 12 can be gradually exposed and released from the sheath 11, and a placement position of the stent graft 50 can be finely adjusted.

The rack 28 and the first gear 271 are configured to be switchable between a meshing state and a separated state. Specifically, when the button 263 of the trigger 26 is pressed, the trigger 26 rotates downward about the trigger connection portion 255c of the gearbox 25, and the first gear 271 moves downward together with the trigger 26. Accordingly, the first gear 271 and the rack 28 enter the separated state. At this time, the first gear shaft 274 to which the first gear 271 is fixed moves along the oblong through-hole 255a provided in the gearbox 25. On the other hand, an engaged state between the front handle 23 and the second gear 272 and an engaged state between the second gear 272 and the third gear 273 are held. Therefore, the gearbox 25, the trigger 26, the gear train 27, and the front handle 23 are movable in the axial direction with respect to the cover main body 22 in which the rack 28 is disposed. Accordingly, after finely adjusting the placement position of the stent graft 50, the stent graft 50 can be released at once by pressing the button 263 to slide to the proximal side S2. When the pressing of the button 263 is released, the trigger 26 returns to an original state by a restoring force of the first spring 24 and the second spring 29, and the rack 28 and the first gear 271 enter the meshing state.

In addition, the gearbox 25 also has the restriction portion 256 that restricts the trigger 26 from being pushed against a biasing force of the first spring 24. On the other hand, the trigger 26 is configured to be movable in the axial direction with respect to the gearbox 25. Specifically, the trigger 26 is connected to the gearbox 25 by the engagement between the oblong bearing hole 261a and the first gear shaft 274 and the engagement between the oblong engagement hole 261b and the trigger connection portion 255c. Therefore, only after the button 263 of the trigger 26 is moved to the proximal side S2, the restriction by the restriction portion 256 is released and the trigger 26 can be pushed. Accordingly, the release of the meshing state between the rack 28 and the first gear 271 due to an erroneous operation can be prevented.

Fig. 8 is an exploded perspective view of the second operating portion 20B. Fig. 9 is a cross-sectional view of the second operating portion 20B. As shown in Figs. 8 and 9, the second operating portion 20B includes the rear handle 31, a handle stopper 32, the fixing terminal 33, the rod guide shaft 34, the Y connector 35, and the like.

The rear handle 31 is an operating member that rotates about the axial direction when the holding wire 142 that is the string-like converting means 14 is pulled out. The rear handle 31 has a cylindrical shaft attachment portion 311 and is fitted to an outer peripheral surface of the rod guide shaft 34. A female thread 312 is formed on an inner peripheral surface of the shaft attachment portion 311. The female thread 312 meshes with a male thread 332 of the fixing terminal 33 and forms a feed screw mechanism together with the male thread 332.

The handle stopper 32 is a bowl-shaped member, is disposed on a proximal side S2 of the rear handle 31, and restricts movement of the rear handle 31 in the axial direction. The handle stopper 32 has a recessed portion 321 to which an end portion of the rod guide shaft 34 on the proximal side S2 is attached. An engagement piece 322 is formed on an inner peripheral surface of the recessed portion 321 so as to protrude in the radial direction. The handle stopper 32 is detachably attached to the rod guide shaft 34. When the handle stopper 32 is detached from the rod guide shaft 34, the restriction of the movement of the rear handle 31 in the axial direction is released, and the rear handle 31 and the fixing terminal 33 can be integrally moved to the proximal side S2.

The fixing terminal 33 is a cylindrical member to which the end portion of the inner rod 12 on the proximal side S2 is fixed. The end portion of the holding wire 142 on the proximal side S2 is also fixed to the fixing terminal 33. For example, in a state in which the holding wire 142 is directed along the inner rod 12, the inner rod 12 and the holding wire 142 are inserted through a rubber block 333, and the rubber block 333 is press-fitted into an insertion hole (reference numerals omitted) of the fixing terminal 33, whereby the inner rod 12 and the holding wire 142 are fixed to the fixing terminal 33. In Fig. 9, the end portion of the inner rod 12 on the proximal side S2 extends through the insertion hole of the fixing terminal 33 to the Y connector 35. In addition, the end portion of the holding wire 142 on the proximal side S2 is pulled out, for example, from an opening (reference numerals omitted) of the fixing terminal 33 and fixed by adhesion.

The fixing terminal 33 has guide pieces 331 directed along the axial direction. The guide pieces 331 are provided, for example, at two points opposing each other in the radial direction. The guide piece 331 is engaged with a guide slit 341 of the rod guide shaft 34. A part of the guide piece 331 is exposed from the guide slit 341 in a state in which the fixing terminal 33 is attached to the rod guide shaft 34. At this portion, the male thread 332 that meshes with the female thread 312 of the rear handle 31 is formed. The number of guide pieces 331 and guide slits 341 is not limited to two, and can be changed as appropriate.

The rod guide shaft 34 is a cylindrical member and guides the movement of the fixing terminal 33 in the axial direction. The guide slit 341 directed along the axial direction is provided over the entire length of the rod guide shaft 34. The guide slit 341 is provided at a position corresponding to the guide piece 331 of the fixing terminal 33. In addition, at the end portion of the rod guide shaft 34 on the proximal side S2, an engagement groove 343 is provided along the circumferential direction starting from the two guide slits 341. One end of the engagement groove 343 communicates with the guide slit 341 and the other end is closed. At a substantially center of the engagement groove 343 in the circumferential direction, a protruding portion 344 that restricts a rotating motion of the handle stopper 32 is provided.

The Y connector 35 is attached to a proximal side S2 of handle stopper 32. The Y connector 35 has insertion holes 351 and 352 that communicate with an inner cavity of the operating means 20. The insertion holes 351 and 352 join on the distal side S1. A guide wire 15 and the restraining string 141 are inserted into the catheter portion 10 through the insertion holes 351 and 352.

The rod guide shaft 34 is fixed to the cover main body 22 so as not to move in the axial direction by fitting a flange 342 provided at the end portion on the distal side S1 into a fixing groove (reference numerals omitted) of the cover main body 22. The fixing terminal 33 is inserted into the rod guide shaft 34 from the proximal side S2 so that the guide piece 331 is inserted into the guide slit 341. In this state, by causing the rear handle 31 to be fitted to the rod guide shaft 34 from the proximal side S2 and tightening the rear handle 31, the female thread 312 of the rear handle 31 and the male thread 332 of the fixing terminal 33 mesh with each other, and the fixing terminal 33 is pushed to an end portion of the rod guide shaft 34 on the distal side S1. The fixing terminal 33 is integrally connected to the rear handle 31 via the feed screw mechanism including the female thread 312 and the male thread 332. On the other hand, the fixing terminal 33 is movable in the axial direction with respect to the rod guide shaft 34.

The end portion of the rod guide shaft 34 on the proximal side S2 (the portion provided with the engagement groove 343) protrudes from a proximal end of the rear handle 31. The handle stopper 32 is attached to this protruding portion. Accordingly, the movement of the rear handle 31 in the axial direction is restricted, and the rear handle 31 is prevented from moving to the proximal side S2 and falling off. In addition, by rotating the handle stopper 32 to detach the handle stopper 32 from the rod guide shaft 34, the restriction of the movement of the rear handle 31 is released.

Specifically, the handle stopper 32 is positioned with respect to the rod guide shaft 34 so that the engagement piece 322 is positioned at an open end of the engagement groove 343. In this state, when the handle stopper 32 is rotated in a direction to be engaged with the engagement groove 343 (a direction opposite to the arrow A in Fig. 8), the engagement piece 322 moves along the engagement groove 343 and climbs over the protruding portion 344. The engagement piece 322 is inserted between a closed end of the engagement groove 343 and the protruding portion 344, so that the handle stopper 32 is held in this state. Since the engagement piece 322 cannot move beyond the closed end of the engagement groove 343, the handle stopper 32 can be rotated only in a direction to be detached about the axial direction. In a case where the handle stopper 32 is detached from the rear handle 31, it is necessary to rotate the handle stopper 32 so that the engagement piece 322 climbs over the protruding portion 344, requiring a predetermined force or more.

In the second operating portion 20B, the rear handle 31 is sandwiched between the cover main body 22 and the handle stopper 32 and cannot move in the axial direction. Therefore, when the rear handle 31 is rotated about the axial direction, the fixing terminal 33 is moved in the axial direction along the guide slit 341 of the rod guide shaft 34 by the feed screw mechanism including the female thread 312 and the male thread 332. Accordingly, the converting means 14 fixed to the fixing terminal 33 advances and retreats in the axial direction.

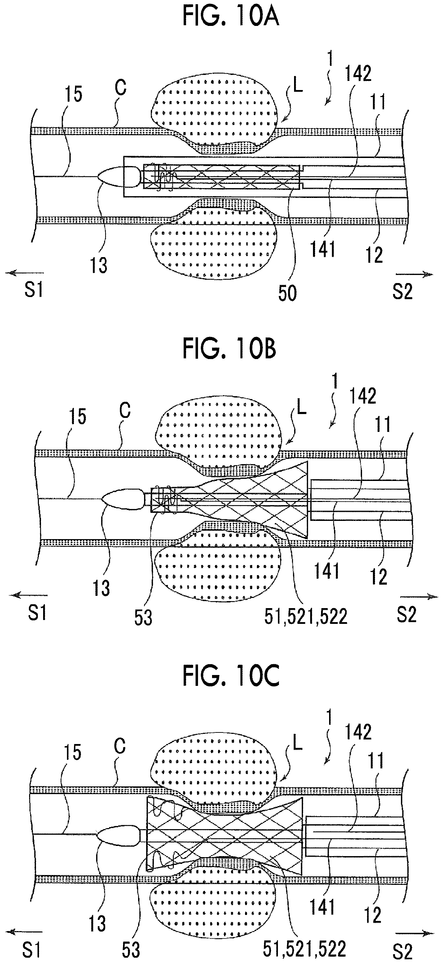

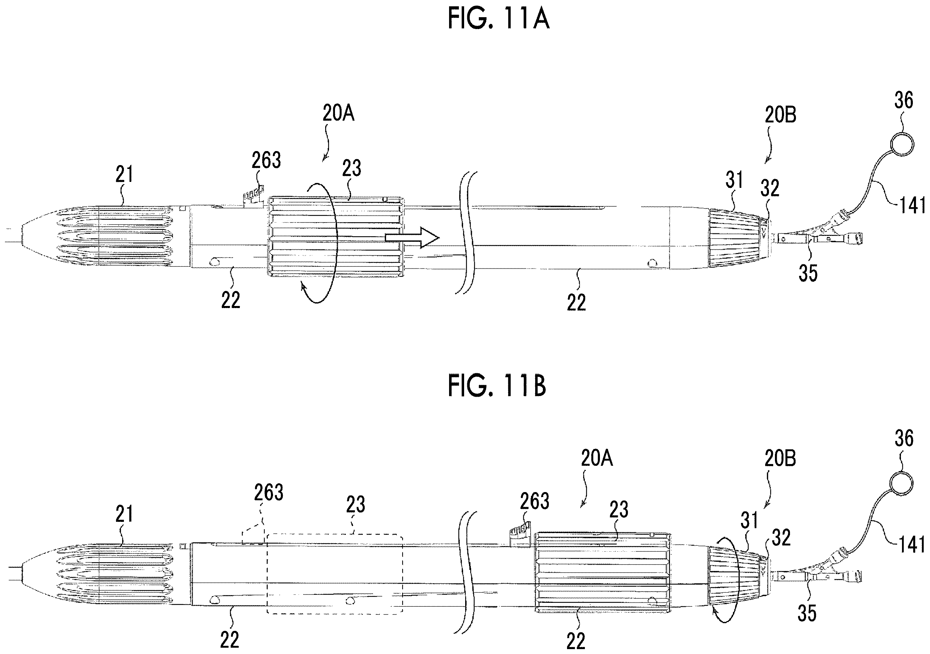

Hereinafter, a process of placing the stent graft 50 in the abdominal aorta C using the placement device 1 will be described below. Figs. 10A to 10C are views showing a change in a state of the stent graft 50 during placement. Figs. 10A to 10C schematically show the stent graft 50. In Figs. 10A to 10C, the distal side S1 is the "upstream side" and the proximal side S2 is the "downstream side" in the blood flow direction. Figs. 11A and 11B are views showing an operation of the operating means 20 during the placement. Fig. 11A shows an operation of releasing the stent graft 50 from the sheath 11 and Fig. 11B shows an operation of pulling out the holding wire 142.

In a case where the stent graft 50 is to be placed at the lesion site L (placement target site) of the abdominal aorta C, the sheath 11 and the inner rod 12 (catheter portion 10) are inserted from the downstream side in the blood flow direction along the guide wire 15 previously introduced into the abdominal aorta C and are positioned so that the stent graft 50 is positioned at the lesion site L (see Fig. 10A).

Next, in the positioned state, the first operating portion 20A is operated to move the sheath 11 to the proximal side S2 (downstream side in the blood flow direction) to release the stent graft 50 from the sheath 11 (see Fig. 10B). An operation of the first operating portion 20A for releasing the sheath 11 is as described above. That is, as shown in Fig. 11A, the operator rotates the front handle 23 of the first operating portion 20A to gradually expose the stent graft 50 mounted on the inner rod 12 from the sheath 11 and release the stent graft 50 while finely adjusting the placement position. Thereafter, the operator presses the button 263 of the trigger 26 to allow the button 263 to slide to the proximal side S2, thereby releasing the stent graft 50 at once. The rear handle 23 and the like are positioned near the second operating portion 20B.

At this time, as shown in Fig. 10B, the main body portion 51, the first branch portion 521 and the second branch portion 522 of the stent graft 50 transition to the expanded state. On the other hand, the bare portion 53 is restrained by the converting means 14 and maintained in the contracted state.

Next, based on the operation of the rear handle 31 of the second operating portion 20B, the holding wire 142 is pulled out, and the restraint of the bare portion 53 of the stent graft 50 by the converting means 14 is released and the bare portion 53 is released. Accordingly, the bare portion 53 also transitions to the expanded state (see Fig. 10C). As shown in Fig. 11B, when the operator rotates the rear handle 31 of the second operating portion 20B, the holding wire 142 moves to the proximal side S2, and the engaged state with the restraining string 141 is released. Since the second operating portion 20B is positioned near the first operating portion 20A after the sheath 11 is moved to the proximal side S2, the operator can intuitively and easily transition to the operation of the second operating portion 20B.

In the above example, after the stent graft 50 is completely released from the sheath 11, the holding wire 142 is pulled out to expand the bare portion 53 of the stent graft 50. However, since the second operating portion 20B is provided independently from the first operating portion 20A, a time for releasing the stent graft 50 from the sheath 11 and a time for releasing the restraint of the bare portion 53 of the stent graft 50 can be individually adjusted. For example, in a state in which only the bare portion 53 of the stent graft 50 is released from the sheath 11, the bare portion 53 may be expanded and thereafter a rest of the stent graft 50 may be released from the sheath 11. Alternatively, for example, the operation of releasing the bare portion 53 of the stent graft 50 from the sheath 11 and the operation of pulling out the holding wire 142 can be performed simultaneously.

As described above, the placement device 1 according to the present embodiment is the placement device 1 for placing the stent graft 50 (tubular indwelling tool) in the abdominal aorta C (biological lumen), and includes the sheath 11 in which the stent graft 50 is accommodated, the string-like holding wire 142 (converting means) that can cause at least a portion (for example, the bare portion 53) of the stent graft 50 in the axial direction to be maintained in the contracted state in a state in which the stent graft 50 is released from the sheath 11 and convert the portion from the contracted state to the expanded state, and the operating means 20 operated to place the stent graft 50 in the abdominal aorta C. The operating means 20 has the first operating portion 20A for releasing the stent graft 50 from the sheath 11 and the second operating portion 20B for releasing the restraint of the portion of the stent graft 50 by the holding wire 142.

According to the placement device 1, the operation of the first operating portion 20A for releasing the stent graft 50 from the sheath 11 and the operation of the second operating portion 20B for releasing the restraint of at least the portion of the stent graft 50 are easily performed using one operating means 20. In addition, the operations of the first operating portion 20A and the second operating portion 20B can be performed independently. Therefore, the placement of the stent graft 50 in the abdominal aorta C can be properly performed.

In addition, the time for releasing the stent graft 50 from the sheath 11 and the time for releasing the restraint of at least the portion of the stent graft 50 can be individually adjusted, so that appropriate and flexible reactions are possible, for example, depending on a placement site, a type of tubular indwelling tool, a case, and the like.

In addition, in the placement device 1, the second operating portion 20B has the fixing terminal 33 (fixing member) to which the holding wire 142 is connected, and the rear handle 31 (operating member) that moves the fixing terminal 33 in the axial direction in response to an operation by the operator.

Accordingly, the string-like holding wire 142 can be moved in the axial direction via the fixing terminal 33 to transition at least the portion of the stent graft 50 maintained in the contracted state to the expanded state, and the stent graft 50 can be properly placed in a placement target site. In addition, since the contracted state of the stent graft 50 is maintained using the string-like holding wire 142, a structure of the device can be simplified.

In addition, in the placement device 1, the rear handle 31 (operating member) is a rotating member, and the second operating portion 20B further has the feed screw mechanism (power transmission portion) that transmits the rotation of the rear handle 31 to the fixing terminal 33.

Accordingly, a space of the rear handle 31 can be saved, and the string-like holding wire 142 can be moved in the axial direction with a stable force via the feed screw mechanism including the female thread 312 of the rear handle 31 and the male thread 332 of the fixing terminal 33.

In addition, in the placement device 1, the second operating portion 20B further has the handle stopper 32 (stopper portion) for restricting the movement of the rear handle 31 (operating member) in the axial direction, and the handle stopper 32 is provided attachable to and detachable from the second operating portion 20B and releases the restriction of the movement of the rear handle 31 by being detached from the second operating portion 20B.

That is, the placement device 1 is the placement device for placing the stent graft 50 (tubular indwelling tool) in the abdominal aorta C (biological lumen), and includes the sheath 11 in which the stent graft 50 is accommodated, the string-like holding wire 142 (converting means) that can cause at least a portion (for example, the bare portion 53) of the stent graft 50 in the axial direction to be maintained in the contracted state in a state in which the stent graft 50 is released from the sheath 11 and convert the portion from the contracted state to the expanded state, and the second operating portion 20B for releasing the restraint of the portion of the stent graft 50 by the holding wire 142, the second operating portion 20B has the rear handle 31 (operating member) that moves the holding wire 142 in the axial direction in response to an operation by the operator and the handle stopper 32 (stopper portion) that restricts the movement of the rear handle 31 in the axial direction, and the handle stopper 32 is provided attachable to and detachable from the second operating portion 20B and releases the restriction of the movement of the rear handle 31 by being detached from the second operating portion 20B.

Accordingly, the rear handle 31 can be prevented from moving in the axial direction and falling off, and as necessary, the handle stopper 32 is detached to release the restriction of the movement of the rear handle 31, so that recovery (pulling out) of the holding wire 142 (converting means) can be properly performed. That is, when the handle stopper 32 is detached from the second operating portion 20B, the restriction of the movement of the rear handle 31 and the fixing terminal 33 to the proximal side S2 is released, and the rear handle 31 and the fixing terminal 33 are movable in the axial direction with respect to the rod guide shaft 34. By sliding the rear handle 31 to the proximal side S2, the holding wire 142 can be easily recovered.

In addition, in the placement device 1, the handle stopper 32 (stopper portion) is configured to be rotatable in only one direction about the axial direction, and to be detachable from the second operating portion 20B by rotating in the one direction.

Accordingly, the restriction of the movement of the rear handle 31 can be easily released by a simple operation of the handle stopper 32 as necessary.

### [Modification Example]

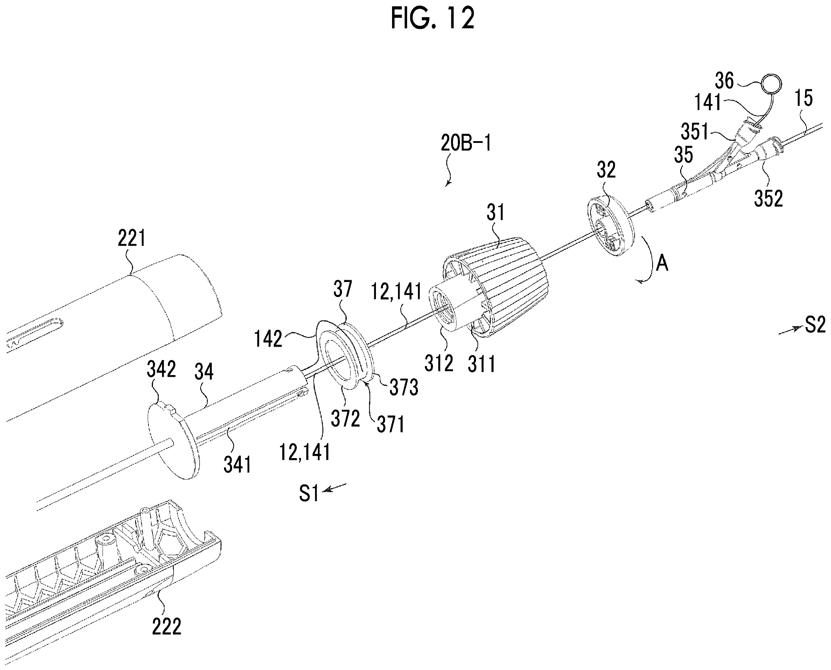

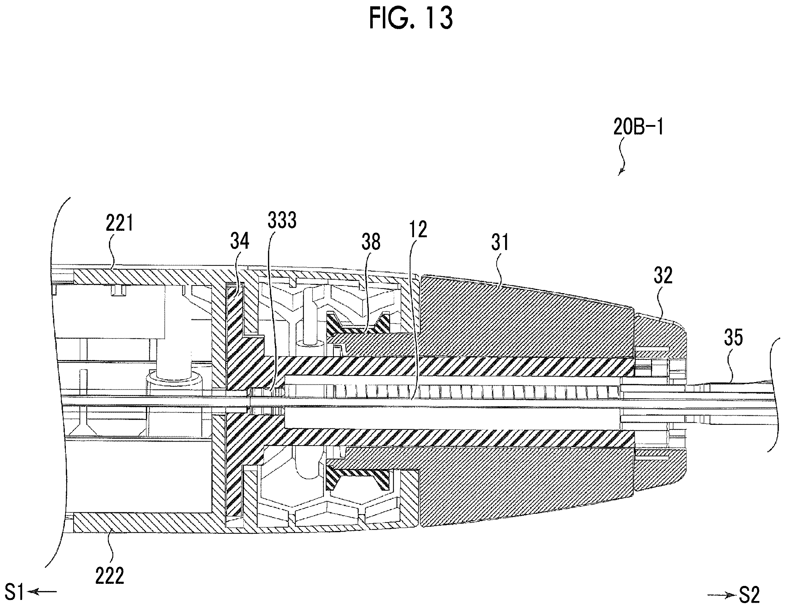

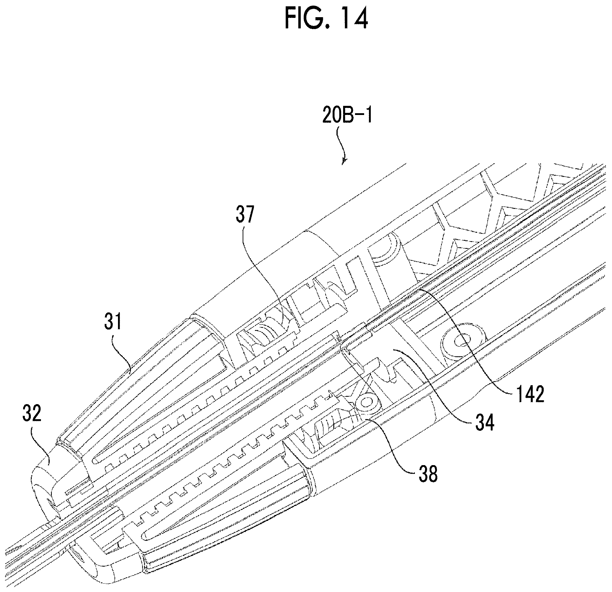

Fig. 12 is an exploded perspective view of a second operating portion 20B-1 according to a modification example. Fig. 13 is a cross-sectional view of the second operating portion 20B-1. Fig. 14 is a partially cutaway cross-sectional perspective view of the second operating portion 20B-1. As shown in Figs. 12 to 14, the second operating portion 20B-1 includes the rear handle 31, the handle stopper 32, a bobbin 37, the rod guide shaft 34, the Y connector 35, and the like.

In the second operating portion 20B-1, instead of the fixing terminal 33 in the embodiment, the bobbin 37 capable of winding the holding wire 142 is provided. Other constituent elements are substantially the same as those of the embodiment, and will be briefly described.

The rear handle 31 is an operating member that rotates about the axial direction when the holding wire 142 that is the string-like converting means 14 is pulled out. The rear handle 31 is fitted into the outer peripheral surface of the rod guide shaft 34 and rotates about the rod guide shaft 34.

The end portions of the inner rod 12, the restraining string 141, and the holding wire 142 on the proximal side S2 are inserted through the rubber block 333, for example. The inner rod 12 is fixed to the rod guide shaft 34 by press-fitting the rubber block 333 into an insertion hole (reference numerals omitted) of the rod guide shaft 34.

The holding wire 142 is held by the rubber block 333 by friction and cannot move in the axial direction unless the rear handle 31 is rotated. That is, the friction between the holding wire 142 and the rubber block 333 restricts the rotation of the rear handle 31. A rotation stopping mechanism (for example, a cam clutch) that mechanically restricts the rotation of the rear handle 31 with respect to the rod guide shaft 34 may be provided.

The end portion of the holding wire 142 on the proximal side S2 is pulled out from the guide slit 341 of the rod guide shaft 34. The holding wire 142 that has been pulled out is wound around columnar folded-back portions 38 provided in the upper cover 221 and the lower cover 222 and is guided to the bobbin 37. By winding the holding wire 142 around the folded-back portions 38, an appropriate tension is applied to the holding wire 142, so that winding defects can be prevented.

The bobbin 37 is a winding portion around which the holding wire 142 is wound. The bobbin 37 has a cylindrical body portion 371 and flange portions 372 and 373 disposed at both axial ends of the body portion 371. The end portion of the holding wire 142 on the proximal side S2 that has been pulled out from the rod guide shaft 34 is fixed to the body portion 371 of the bobbin 37 by, for example, adhesion.

The bobbin 37 is fixed to an outer peripheral surface of the shaft attachment portion 311 of the rear handle 31 by, for example, adhesion. The bobbin 37 is fitted to the outer peripheral surface of the rod guide shaft 34 together with the rear handle 31. The bobbin 37 rotates together with the rear handle 31 along a rotating motion of the rear handle 31. As the bobbin 37 rotates, the holding wire 142 is directly wound around the body portion 371 and pulled out.

As described above, the second operating portion 20B-1 according to the modification example has the bobbin 37 (winding portion) around which the holding wire 142 (converting means) is wound and the rear handle 31 (operating member) that rotates the bobbin 37 in response to a rotating operation by the operator.

In a case where the holding wire 142 is pulled out to the feed screw mechanism provided in the rear handle 31 and the fixing terminal 33 as in the embodiment, a movement distance of the holding wire 31 for each rotation of the rear handle 31 is short. Contrary to this, in the modification example in which the holding wire 142 is directly wound around the bobbin 37, the holding wire 142 is wound by a rotation amount of the rear handle 31, so that the holding wire 142 can be easily pulled out. Therefore, the stent graft 50 restrained in the contracted state can be immediately released and expanded.

In addition, unlike the feed screw mechanism, there is no restriction on the movement distance. Therefore, a length of the shaft attachment portion 311 of the rear handle 31 to which the bobbin 37 is attached can be shortened, and a reduction in size of the placement device 1 can be achieved. In addition, it is possible to lengthen a length of the holding wire 142 and lengthen a restraint length of the stent graft 50. Therefore, restraining a large stent graft 50 can be easily handled.

In the above description, the bobbin 37 is directly fixed to the rear handle 31. However, a power transmission member such as a gear may be interposed between the rear handle 31 and the bobbin 37 so that a rotation amount of the bobbin 37 with respect to the rotation amount of the rear handle 31 can be adjusted. In addition, the winding portion around which the holding wire 142 is wound may be formed integrally with the rear handle 31 as one molded component.

Although the invention made by the inventor of the present invention has been specifically described above based on the embodiments, the present invention is not limited to the above embodiments, and can be changed without departing from the scope of the invention.

For example, the converting means 14 including the holding wire 142 and the restraining string 141 shown in the embodiment is an example of string-like converting means, and other configurations can be applied. For example, the contracted state of the stent graft 50 may be restrained by winding a string-like member around the outer peripheral surface of the stent graft 50, and the restrained state may be released by pulling out the string-like member by operating the second operating portion 20B.

In addition, in the embodiment, the case where the bare portion 53 positioned at an end portion of the stent graft 50 on the distal side S1 is restrained in a contracted state by the converting means 14 has been described, but the portion restrained by the converting means 14 may be a center portion of the stent graft 50 in the axial direction or an end portion of the stent graft 50 on the proximal side S2.

In addition, the present invention is not limited to the stent graft 50 described in the embodiments, and can be applied to a placement device for placing a tubular indwelling tool to be placed in a biological lumen such as a digestive system lumen. The tubular indwelling tool placed by the placement device includes not only the stent graft but also those in which a tip end portion of a device is temporarily placed in a biological lumen, such as an anti-adhesion material and a thrombus removal device (refer to JP-A-2015-107301).

The embodiments disclosed herein are merely examples in all respects, and should not be considered restrictive. The scope of the present invention is represented by the appended, not by the above description, and is intended to include all modifications within the meaning and the scope which are equivalent to those of the appended claims.

The disclosure contents of the specification, drawings, and abstract contained in the Japanese application of Japanese Patent Application No. 2021-057476 filed on March 30, 2021 and the Japanese application of Japanese Patent Application No. 2021-059197 filed on March 31, 2021 are all incorporated herein by reference.

### Reference Signs List

1: placement device
10: catheter portion
11: sheath
12: inner rod
14: converting means
141: restraining string
142: holding wire
20: operating means
20A: first operating portion
20B: second operating portion
31: rear handle (operating member, rotating member)
312: female thread (power transmission portion)
32: handle stopper (stopper portion)
33: fixing terminal
332: male thread (power transmission portion)
50: stent graft (tubular indwelling tool)
C: abdominal aorta (biological lumen)

## Claims

1. A placement device for placing a tubular indwelling tool in a biological lumen, the placement device comprising:
a sheath in which the tubular indwelling tool is accommodated;
string-like converting means that causes at least a portion of the tubular indwelling tool in an axial direction to be maintained in a contracted state in a state in which the tubular indwelling tool is released from the sheath and is able to convert the portion from the contracted state to an expanded state; and
operating means operated to place the tubular indwelling tool in the biological lumen,
wherein the operating means has a first operating portion for releasing the tubular indwelling tool from the sheath, and a second operating portion for releasing restraint of the portion of the tubular indwelling tool by the converting means.

2. The placement device according to claim 1,
wherein the second operating portion has
a fixing member to which the converting means is connected, and
an operating member that moves the fixing member in the axial direction in response to an operation by an operator.

3. The placement device according to claim 2,
wherein the operating member is a rotating member, and
the second operating portion further includes a power transmission portion that transmits rotation of the rotating member to the fixing member.

4. The placement device according to claim 1,
wherein the second operating portion has
a winding portion around which the converting means is wound, and
an operating member that rotates the winding portion in response to a rotating operation by an operator.

5. The placement device according to any one of claims 2 to 4,
wherein the second operating portion further includes a stopper portion that restricts movement of the operating member in the axial direction, and
the stopper portion is provided attachable to and detachable from the second operating portion, and is detached from the second operating portion to release restriction of the movement of the operating member.

6. The placement device according to claim 5,
wherein the stopper portion is rotatable in only one direction about the axial direction, and is configured to be detachable from the second operating portion by rotating in the one direction.
